# EUROPÄISCHE PATENTSCHRIFT

(11) **EP 0 716 685 B1**
(45) Veröffentlichungstag und Bekanntmachung des Hinweises auf die Patenterteilung: **15.10.1997**
(21) Anmeldenummer: 94926863.5
(22) Anmeldetag: 22.08.1994
(51) Int. Cl.: C11D 3/386, C12N 9/98

(54) **MEHRENZYMGRANULAT**
MULTIPLE ENZYME GRANULATE
GRANULE CONTENANT PLUSIEURS ENZYMES

(30) Priorität: 01.09.1993 DE 4329463
(43) Veröffentlichungstag der Anmeldung: 19.06.1996
(73) Patentinhaber: Henkel Kommanditgesellschaft auf Aktien, 40191 Düsseldorf (DE)
(72) Erfinder: PAATZ, Kathleen, D-40589 Düsseldorf (DE); RÄHSE, Wilfried, D-40589 Düsseldorf (DE); PICHLER, Werner, A-6250 Kundl (AT); UPADEK, Horst, D-40883 Ratingen (DE); KÜHNE, Norbert, D-42781 Haan (DE)
(86) Internationale Anmeldenummer: EP9402779
(87) Internationale Veröffentlichungsnummer: WO9506709

(56) Entgegenhaltungen:
- EP-A- 0 304 332
- WO-A-90/09440
- WO-A-92/11347
- CHEMICAL ABSTRACTS, Band 105, Nr. 26, 29 Dezember 1986, Columbus, OH (US); Seite 119, AN 228951h

## Beschreibung

Die Erfindung betrifft ein Enzymgranulat, das mindestens zwei verschiedene Enzyme enthält, ein Verfahren zu seiner Herstellung und die Verwendung des Granulats in festen oder flüssigen Wasch- und Reinigungsmitteln.

Enzyme, insbesondere Proteasen, finden ausgedehnte Verwendung in Wasch-, Waschhilfs- und Reinigungsmitteln. Üblicherweise kommen die Enzyme dabei nicht als Konzentrate, sondern in Mischungen mit einem Verdünnungs- und Trägermaterial zum Einsatz. Mischt man solche Enzymzubereitungen üblichen Waschmitteln bei, so kann beim Lagern ein erheblicher Abbau der Enzymaktivität eintreten, insbesondere wenn bleichaktive Verbindungen zugegen sind. Das Aufbringen der Enzyme auf Trägersalze unter gleichzeitiger Granulation gemäß der deutschen Offenlegungsschrift DT 16 17 190 beziehungsweise durch Aufkleben mit nichtionischen Tensiden gemäß der deutschen Offenlegungsschrift DT 16 17 118 oder wäßrigen Lösungen von Celluloseethern gemäß der deutschen Offenlegungschrift DT 17 87 568 führt nicht zu einer nennenswerten Verbesserung der Lagerstabilität, da sich die empfindlichen Enzyme in solchen Aufmischungen in der Regel auf der Oberfläche der Trägersubstanz befinden. Zwar kann die Lagerstabilität der Enzyme wesentlich erhöht werden, wenn man die Enzyme mit dem Trägermaterial umhüllt beziehungsweise in dieses einbettet und anschließend durch Extrudieren, Pressen und Spheronisieren in die gewünschte Partikelform überführt, wie zum Beispiel in der deutschen Patentschrift DE 16 17 232, der deutschen Offenlegungsschrift DT 20 32 768 und den deutschen Auslegeschriften DE 21 37 042 und DE 21 37 043 beschrieben. Derartige Enzymzubereitungen besitzen jedoch nur mangelhafte Löslichkeitseigenschaften. Die ungelösten Partikel können sich im Waschgut verfangen und dieses verunreinigen bzw. sie werden ungenutzt in das Abwasser überführt. Aus der deutschen Offenlegungsschrift DT 18 03 099 bekannte Einbettungsmittel, die aus einem Gemisch fester Säuren beziehungsweise saurer Salze und Carbonaten beziehungsweise Bicarbonaten bestehen und bei Wasserzusatz zerfallen, verbessern zwar das Lösungsvermögen, sind aber ihrerseits sehr empfindlich gegen Feuchtigkeit und erfordern daher zusätzliche Schutzmaßnahmen. Ein weiterer Nachteil der vorgenannten Zubereitung ist darin zu sehen, daß die Enzyme nur in Form trockener Pulver verarbeitet werden können. Die üblicherweise bei der Enzymherstellung anfallenden Fermentbrühen lassen sich in dieser Form nicht einsetzen, sondern müssen zuvor entwässert werden.

Aus der europäischen Patentschrift EP 168 526 sind Enzymgranulate bekannt, die in Wasser quellfähige Stärke, Zeolith und wasserlösliches Granulierhilfsmittel enthalten. In diesem Dokument wird ein das obengenannte Problem umgehendes Herstellungsverfahren für derartige Formulierungen vorgeschlagen, das im wesentlichen darin besteht, eine von unlöslichen Bestandteilen befreite Fermenterlösung aufzukonzentrieren, mit den genannten Zuschlagstoffen zu versetzten und das entstandene Gemisch zu granulieren. Das Verfahren mit dem dort vorgeschlagenen Zuschlagstoffgemisch wird vorteilhaft mit Fermentationslösungen durchgeführt, die auf einen relativ hohen Trockensubstanzgehalt, beispielsweise 55 Gew.-%, aufkonzentriert worden sind.

Aus der internationalen Patentanmeldung WO 92/11347 sind Enzymgranulate zum Einsatz in körnigen Wasch- und Reinigungsmitteln bekannt, die 2 Gew.-% bis 20 Gew.-% Enzym, 10 Gew.-% bis 50 Gew.-% quellfähige Stärke, 5 Gew.-% bis 50 Gew.-% wasserlösliches organisches Polymer als Granulierhilfsmittel, 10 Gew.-% bis 35 Gew.-% Getreidemehl und 3 Gew.-% bis 12 Gew.-% Wasser enthalten. Durch derartige Zuschlagstoffe wird die Enzymverarbeitung ohne größere Aktivitätsverluste möglich und auch die Lagerbeständigkeit der Enzyme in den Granulaten ist zufriedenstellend.

Wie anhand der genannten Dokumente beispielhaft geschildert, existiert ein breiter Stand der Technik auf dem Gebiet der Herstellung von granularen Enzymzubereitungen, so daß dem Fachmann verschiedene Möglichkeiten zur teilchenförmigen Konfektionierung von einzelnen Enzymen zur Verfügung stehen. Die genannten Methoden versagen jedoch, wenn es darum geht, zwei oder mehrere Enzyme, welche miteinander reagieren können, in dasselbe Granulat einzuarbeiten. Insbesondere stellt sich dieses Problem im Zusammenhang mit Protease, welche als proteinabbauendes Enzym naturgemäß in der Lage ist, ein gleichzeitig vorhandenes zweites und/oder weiteres Enzym zu zersetzen. Wenn diese Zersetzung bei der Herstellung und/oder während der Lagerung der Enzymgranulate eintritt, ist die Wirkung des zweiten und/oder weiteren Enzyms unter Anwendungsbedingungen nicht mehr gewährleistet.

Auch zur Lösung dieses Problems gibt es Ansätze im Stand der Technik. So wird in der internationalen Patentanmeldung WO 90/09440 ein Zweienzymgranulat dadurch hergestellt, daß man einen protease- und cellulosehaltigen Kern mit insgesamt 10 Schichten (abwechselnd Stearinsäure-/Palmitinsäure-glycerid und Kaolin) überzieht, wobei in den Beispielen die Menge des Schutzüberzugsmaterials diejenige des Kerns übersteigt, anschließend ein Gemisch aus einem zweiten Enzym, einem Bindemittel, einem Füllmaterial und einem Granulierhilfsmittel aufbringt und schließlich mit einer äußeren Coatingschicht umhüllt. Ein derartiges Herstellverfahren ist wegen des hohen Bedarfs an Trennmaterial zwischen dem enzymhaltigen Kern und der das zweite Enzym enthaltenden, weiter außen liegenden Schicht ungünstig.

Aus der europäischen Patentanmeldung EP 304 332 ist bekannt, daß man ein enzymhaltiges Basisgranulat mit pulverförmigen Komponenten, welche ein zweites Enzym umfassen, umhüllen kann. Diese Art der Herstellung von Mehrenzymgranulaten führt jedoch zu mangelnder Stabilität des zweiten, in der äußeren Schicht befindlichen Enzyms, das überdies nachteiligerweise zuvor in feinteiliger Pulverform vorliegend zubereitet werden muß.

Es bestand daher die Aufgabe, ein möglichst einfaches Herstellungsverfahren für teilchenförmige Enzymzubereitungen zu entwickeln, welche mindestens zwei verschiedene, miteinander reagierende Enzyme enthalten, wobei die Enzyme ohne Aktivitätsverlust in das Mehrenzymgranulat eingearbeitet werden müssen und dort lagerstabil verbleiben sollen. Dies gelang überraschenderweise im wesentlichen durch den Einsatz zweier separat hergestellter, jeweils ein Enzym enthaltender Granulate mit unterschiedlichen Korngrößen in einem anschließenden Cogranulationsschritt, in dem das kleinere Granulat agglomerierend das größere Granulat umhüllt.

Die Erfindung betrifft demgemäß ein Verfahren zur Herstellung von Enzymgranulaten mit mittleren Korngrößen von 1 mm bis 2,2 mm, welche mindestens zwei verschiedene Enzyme enthalten, gekennzeichnet durch Extrudieren eines durch Vermischen einer gegegebenfalls von unlöslichen Bestandteilen befreiten und aufkonzentrierten Fermentationsbrühe eines ersten Enzyms mit organischem und/oder anorganischem Trägermaterial und gegebenenfalls Granulierhilfsmittel als Zuschlagstoffen entstandenen Enzym-Vorgemischs, gegebenenfalls Sphäronisierung des Extrudats in einem Rondiergerät, Trocknung bis zu einem Restfeuchtegehalt von 4 Gew.-% bis 10 Gew.-%, falls das Extrudat vorher höhere Wassergehalte aufweist, Vermischen mit einem teilchenförmig konfektioniertem zweiten Enzym und gegebenenfalls weiteren teilchenförmig konfektionierten Enzymen, wobei weniger als 25 Gew.-% der Teilchen einen Durchmesser unter 0,5 mm aufweisen, unter Agglomerationsbedingungen, wobei gegebenenfalls ein Bindemittel zum Einsatz kommt, und gegebenenfalls Aufbringen eines Farbstoff oder Pigment enthaltenden Überzugs, wobei die mittlere Teilchengröße des das erste Enzym enthaltenden Extrudatkerns das 1,1- bis 3-fache, vorzugsweise das 1,1- bis 2,5-fache und insbesondere das 1,3- bis 2-fache derjenigen des zweiten oder weiteren teilchenförmig konfektionierten Enzyms beträgt. Bei diesen Größenverhältnissen sind die die Kontaktflächen zwischen den zu agglomerierenden Körnern bezogen auf deren Gesamtoberfläche besonders günstig.

Ein weiterer Gegenstand der Erfindung ist ein für die Einarbeitung in insbesondere teilchenförmige Wasch- oder Reinigungsmittel geeignetes Enzymgranulat, enthaltend Enzym und anorganisches und/oder organisches Trägermaterial sowie gegebenenfalls Granulierhilfsmittel, welches dadurch gekennzeichnet ist, daß es aus einem extrudierten, ein erstes Enzym enthaltenden Kern, an den Teilchen, welche ein zweites Enzym und gegebenenfalls Teilchen, welche weitere Enzyme enthalten, agglomeriert sind, wobei weniger als 25 Gew.-% der Teilchen einen Durchmesser unter 0,5 mm aufweisen, und einem aufgesprühten Außencoating besteht, wobei vorzugsweise die mittlere Teilchengröße der zuvor separat hergestellten, ein zweites Enzym oder die weiteren Enzyme enthaltenden Teilchen das 0,5- bis 0,9-fache derjenigen des Extrudatkerns beträgt.

Vorzugsweise handelt es sich bei dem Enzym im Granulatkern um Protease und bei dem in den separat hergestellten, kleineren Teilchen, welche an das Extrudat agglomerieren, enthaltenen Enzym beziehungsweise bei den Enzymen, falls mehrere verschiedene kleinere Teilchen eingesetzt werden, um Amylase, Lipase, Cellulase und/oder Oxidase, obwohl im erfindungsgemäßen Verfahren auch zum Erfolg führt, wenn die Protease in den aufzuagglomerierenden kleineren Teilchen enthalten ist und im Extrudatkern eines der genannten anderen Enzyme vorhanden ist.

In einer bevorzugten Ausführungsform enthält das erfindungsgemäße Enzymgranulat in seinem durch Extrusion hergestellten Kern Protease und in den daran agglomerierten kleineren Teilchen Lipase, wobei letztere vorzugsweise aus Humicola lanuginosa, wie beispielsweise in den europäischen Patentanmeldungen EP 258 068, EP 305 216 und EP 341 947 beschrieben, aus Bacillus-Arten, wie beispielsweise in der internationalen Patentanmeldung WO 91/16422 oder der europäischen Patentanmeldung EP 384 717 beschrieben, aus Pseudomonas-Arten, wie beispielsweise in den europäischen Patentanmeldungen EP 468 102, EP 385 401, EP 375 102, EP 334 462, EP 331 376, EP 330 641, EP 214 761, EP 218 272 oder EP 204 284 oder der internationalen Patentanmeldung WO 90/10695 beschrieben, aus Fusarium-Arten, wie beispielsweise in der europäischen Patentanmeldung EP 130 064 beschrieben, aus Rhizopus-Arten, wie beispielsweise in der europäischen Patentanmeldung EP 117 553 beschrieben, oder aus Aspergillus-Arten, wie beispielsweise in der europäischen Patentanmeldung EP 167 309 beschrieben, gewinnbar ist. Geeignete Lipasen sind beispielsweise unter den Namen Lipolase^{(R)}, Lipozym^{(R)}, Amano^{(R)}-Lipase, Toyo-Jozo^{(R)}-Lipase, Meito^{(R)}-Lipase, und Diosynth^{(R)}-Lipase im Handel erhältlich.

Besonders für den Einsatz in Geschirrspülmitteln, insbesondere zum maschinellen Einsatz, geeignet ist ein erfindungsgemäßes Mehrenzymgranulat, welches in seinem Extrudatkern Protease und in den aufagglomerierten kleineren Teilchen Amylase enthält. Auch die umgekehrte Zusammensetzung, das heißt Amylase im Extrudatkern und Protease in den kleineren Teilchen, ist möglich. Geeignete Amylasen sind beispielsweise unter den Namen Maxamyl^{(R)} und Termamyl^{(R)} handelsüblich.

In einer weiteren bevorzugten Ausführungsform enthält das erfindungsgemäße Enzymgranulat zwei verschiedenartige kleinere Teilchen an einem protease-haltigen Extrudatkern agglomeriert, wobei die kleineren Teilchen entweder lipasehaltig oder cellulasehaltig sind. Derartige erfindungsgemäße Enzymgranulate können durch zeitlich nachfolgendes Aufagglomerieren erst eines und dann des anderen kleineren Enzymteilchens hergestellt werden. Wegen seiner Einfachheit bevorzugt ist allerdings das Vermischen der beiden kleineren Enzymteilchen mit dem Extrudatkern und das gleichzeitige Aufagglomerieren beider kleinerer Enzymteilchen auf den Extrudatkern.

Als im Extrudatkern des erfindungsgemäßen Enzymgranulats enthaltene Enzyme (erstes Enzym) kommen in erster Linie die aus Mikroorganismen, wie Bakterien oder Pilzen, gewonnenen Proteasen, Lipasen, Amylasen, Cellulasen und Oxidasen in Frage, wobei Proteasen, insbesondere die von Bacillus-Arten erzeugten, bevorzugt sind. Sie können in bekannter Weise durch Fermentationsprozesse aus geeigneten Mikroorganismen gewonnen werden, die zum Beispiel in den deutschen Offenlegungsschriften DE 19 40 488, DE 20 44 161, DE 22 01 803 und DE 21 21 397, den US-amerikanischen Patentschriften US 3 632 957 und US 4 264 738, der europäischen Patentanmeldung EP 006 638 sowie der internationalen Patentanmeldung WO 91/2792 beschrieben sind.

Das erste Enzym ist in den erfindungsgemäßen Granulaten vorzugsweise in Mengen von 4 Gew.-% bis 20 Gew.-% enthalten. Falls es sich bei dem erfindungsgemäßen Enzymgranulat um eine proteasehaltige Formulierung handelt, beträgt die Proteaseaktivität vorzugsweise 70 000 Proteaseeinheiten (PE, bestimmt nach der in Tenside 7 (1970), 125 beschriebenen Methode) bis 350 000 PE, insbesondere 120 000 PE bis 300 000 PE, pro Gramm Enzymgranulat.

Die Herstellung der das erste Enzym enthaltenden Teilchen, der sogenannten Extrudatkerne, an welche die kleineren, das zweite Enzym enthaltenden Teilchen agglomerieren, beinhaltet einen Extrusionsschritt, wie unten beschrieben.

Die Herstellung der kleineren Teilchen, welche das zweite Enzym enthalten, ist nicht auf ein besonderes Verfahren beschränkt, sofern darauf geachtet wird, daß das Herstellungsverfahren Enzymgranulate mit mittleren Teilchengrößen ergibt, wie sie für das erfindungsgemäße Verfahren erforderlich sind. Möglich ist demnach, auch das das zweite Enzym enthaltende kleinere Teilchen durch ein Extrusionsverfahren, wie beispielsweise in der internationalen Patentanmeldung WO 92/11347 oder der europäischen Patentschrift EP 168 526 beschrieben, herzustellen. Vorzugsweise stellt man die das zweite Enzym enthaltenden Teilchen durch Aufbaugranulation aus einem anorganischen und/oder organischen Trägermaterial und wäßriger Enzymlösung her. Ein solches Vorgehen unter Verwendung von anorganischem Salz und Cellulosefasern im Trägermaterial und Wasser und/oder wachsartiger Substanz als Bindemittel ist beispielsweise in der deutschen Patentschrift DE 27 30 481 beschrieben. Auch die neben dem zweiten Enzym enthaltenen weiteren Bestandteile der kleineren Teilchen sind nicht kritisch, obwohl für den bevorzugten Einsatz des erfindungsgemäßen Enzymgranulats in Wasch- und Reinigungsmitteln übliche Inhaltsstoffe solcher Mittel oder zumindest mit diesen verträgliche Stoffe bevorzugt sind. Vorzugsweise enthalten die kleineren, das zweite Enzym enthaltenden Teilchen anorganisches Salz, insbesondere Alkalisulfat und/oder -chlorid, in Mengen von, jeweils bezogen auf gesamtes kleineres Teilchen, 30 Gew.-% bis 80 Gew.-%, faser- oder pulverförmige Cellulose in Mengen von 2 Gew.-% bis 40 Gew.-%, Bindemittel, insbesondere Dextrose, Saccharose, Polyvinylalkohol und/oder Polyvinylpyrrolidon, in Mengen von 0,1 Gew.-% bis 15 Gew.-%.

Als Trägermaterialien für das im Extrudatkern enthaltene erste Enzym sind im Prinzip alle organischen oder anorganischen pulverförmigen Substanzen brauchbar, welche die zu granulierenden Enzyme nicht oder nur tolerierbar wenig zerstören oder desaktivieren und unter Extrusions- und den nachfolgenden Granulationsbedingungen stabil sind. Zu derartigen Substanzen gehören beispielsweise Stärke, Getreidemehl, Cellulosepulver, Alkalialumosilikat, insbesondere Zeolith, Schichtsilikat, zum Beispiel Bentonit oder Smectit, und wasserlösliche anorganische oder organische Salze, zum Beispiel Alkalichlorid, Alkalisulfat, Alkalicarbonat oder Alkaliacetat, wobei Natrium oder Kalium die bevorzugten Alkalimetalle sind. Bevorzugt wird ein Trägermaterialgemisch aus in Wasser quellfähiger Stärke, Getreidemehl und gegebenenfalls Cellulosepulver sowie Alkalicarbonat eingesetzt.

Bei der in Wasser quellfähigen Stärke handelt es sich vorzugsweise um Maisstärke, Reisstärke, Kartoffelstärke oder Gemische aus diesen, wobei der Einsatz von Maisstärke besonders bevorzugt ist. Quellfähige Stärke ist in den Extrudatkernenen vorzugsweise in Mengen von 20 Gew.-% bis 50 Gew.-%, insbesondere von 25 Gew.-% bis 45 Gew.-%, jeweils bezogen auf Extrudatkern, enthalten. Dabei beträgt die Summe der Mengen der quellfähigen Stärke und des Mehls vorzugsweise nicht über 80 Gew.-%, insbesondere 32 Gew.-% bis 65 Gew.-%.

Bei dem geeigneten Getreidemehl handelt es sich insbesondere um ein aus Weizen, Roggen, Gerste oder Hafer herstellbares Produkt oder um ein Gemisch dieser Mehle, wobei Vollkornmehle bevorzugt sind. Unter einem Vollkornmehl wird im Rahmen der Erfindung ein nicht voll ausgemahlenes Mehl verstanden, das aus ganzen, ungeschälten Körnern hergestellt worden ist oder zumindest überwiegend aus einem derartigen Produkt besteht, wobei der Rest aus voll ausgemahlenem Mehl beziehungsweise Stärke besteht. Vorzugsweise werden handelsübliche Weizenmehl-Qualitäten, wie Type 450 oder Type 550, eingesetzt. Auch die Verwendung von Mehlprodukten der zu vorgenannten quellfähigen Stärken führenden Getreidearten ist möglich, wenn darauf geachtet wird, daß die Mehle aus den ganzen Körnern hergestellt worden sind. Durch die Mehlkomponente des Zuschlagstoffgemisches wird bekanntermaßen eine wesentliche Geruchsreduzierung der Enzymzubereitung erreicht, welche die Geruchsverminderung durch die Einarbeitung gleicher Mengen entsprechender Stärkearten bei weitem übertrifft. Derartiges Getreidemehl ist in den Extrudatkernen für die erfindungsgemäßen Enzymextrudate vorzugsweise in Mengen von 10 Gew.-% bis 35 Gew.-%, insbesondere von 15 Gew.-% bis 25 Gew.-%, jeweils bezogen auf Extrudatkern, enthalten.

Als zusätzliche Bestandteile des Trägermaterials für das im Extrudatkern enthaltene erste Enzym können Granulierhilfsmittel vorhanden sein, zu denen beispielsweise Cellulose- oder Stärkeether, wie Carboxymethylcellulose, Carboxymethylstärke, Methylcellulose, Hydroxyethylcellulose, Hydroxypropylcellulose sowie entsprechende Cellulosemischether, Gelatine, Casein, Traganth, Maltodextrose, Saccharose, Invertzucker, Glukosesirup oder andere in Wasser lösliche beziehungsweise gut dispergierbare Oligomere oder Polymere natürlichen oder synthetischen Ursprungs verwendet werden. Zu den synthetischen wasserlöslichen Polymeren sind Alkyl- bzw. Alkenylpolyethoxylate, Polyethylenglykole, Polyacrylate, Polymethacrylate, Copolymere der Acrylsäure mit Maleinsäure oder vinylgruppenhaltigen Verbindungen, ferner Polyvinylalkohol, teilverseiftes Polyvinylacetat und Polyvinylpyrrolidon zu rechnen. Soweit es sich bei den vorgenannten Granulierhilfsmitteln um solche mit Carboxylgruppen handelt, liegen diese normalerweise in Form ihrer Alkalisalze, insbesondere ihrer Natriumsalze vor. Derartige Granulierhilfsmittel können in den erfindungsgemäß geeigneten Enzymextrudaten in Mengen bis zu 10 Gew.-%, insbesondere von 0,5 Gew.-% bis 8 Gew.-%, jeweils bezogen auf Extrudatkern, enthalten sein. Polyethylenglykole werden vorzugsweise unter denjenigen mit mittleren Molmassen von 200 bis 600 ausgewählt. Höhermolekulare Polyethylenglykole, das heißt solche mit einem mittleren Molekulargewicht über 1000, sind zwar als synthetische wasserlösliche Polymere mit guter stauboindender Wirkung brauchbar, doch können gerade die höhermolekularen Polyethylenglykole oft eine unerwünschte Erhöhung der Auflösezeit des Extrudatkerns unter Anwendungsbedingungen in Wasser bewirken, so daß diese Substanzen im Extrudatkern des erfindungsgemäßen Enzymgranulats vorzugsweise fehlen. Der Substitutionsgrad bei vorzugsweise eingesetzten Carboxymethylcellulosen liegt im Bereich von 0,8 bis 0,95, da bei deren Einsatz besonders feste Granulatkörner erhalten werden beziehungsweise geringere Mengen erforderlich sind, um eine bestimmte Granulatfestigkeit zu erreichen, als bei Einsatz von Cellulose mit niedrigerem Substitutionsgrad. AuBerdem kann durch den Einsatz der genannten höhersubstituierten Carboxymethylcellulose bei der Herstellung der Granulate im Extrusionsschritt ein höherer Durchsatz durch den Extruder erreicht werden. Unter dem Substitutionsgrad der Carboxymethylcellulose ist die Zahl der veretherten, eine Carboxymethylgruppe tragenden Sauerstoffatome pro Saccharid-Monomer der Cellulose zu verstehen.

Der Extrudatkern des erfindungsgemäßen Enzymgranulats enthält in einer bevorzugten Ausführungsform 4 Gew.-% bis 20 Gew.-%, berechnet als Trockensubstanz, Protease, Lipase, Amylase und/oder Cellulase, 70 Gew.-% bis 90 Gew.-% anorganisches und/oder organisches Trägermaterial und 5 Gew.-% bis 50 Gew.-% carboxymethylcellulosehaltiges Granulierhilfsmittel sowie als Rest auf 100 Gew.-% Wasser.

Als Granulierhilfsmittel enthält der Extrudatkern des erfindungsgemäßen Enzymgranulats vorzugsweise ein Gemisch aus, jeweils bezogen auf Extrudatkern, 0,1 Gew.-% bis 10 Gew.-%, insbesondere 0,5 Gew.-% bis 5 Gew.-% Carboxymethylcellulose sowie 0,1 Gew.-% bis 10 Gew.-%, insbesondere 0,5 Gew.-% bis 4 Gew.-% Polyethylenglykol mit einer mittleren Molmasse von 200 bis 600 und/oder eines Alkyl- bzw. Alkenylpolyethoxylats gemäß Formel (I),

R-(OCH₂CH₂)ₙ-OH (I)

in der R einen geradkettigen oder verzweigten Alkyl- oder Alkenylrest mit bis zu 3 C-C-Doppelbindungen mit 10 bis 22, insbesondere 12 bis 18 C-Atomen und der mittlere Ethoxylierungsgrad n eine Zahl von 30 bis 80 bedeutet.

Zur Herstellung der erfindungsgemäßen Enzymgranulate geht man vorzugsweise von Fermentbrühen aus, die von unlöslichen Begleitstoffen, beispielsweise durch Mikrofiltration, befreit werden. Die Mikrofiltration wird dabei vorzugsweise als Querstrom-Mikrofiltration unter Verwendung poröser Rohre mit Mikroporen größer 0,1 µm, Fließgeschwindigkeiten der Konzentratlösung von mehr als 2 m/s und einem Druckunterschied zur Permeatseite von unter 5 bar durchgeführt, wie zum Beispiel in der europäischen Patentanmeldung EP 200 032 beschrieben. Anschließend wird das Mikrofiltrationspermeat vorzugsweise durch Ultrafiltration, gegebenenfalls mit anschließender Vakuumeindampfung, aufkonzentriert. Die Aufkonzentration kann dabei, wie in der internationalen Patentanmeldung WO 92/11347 beschriebnen, so geführt werden, daß man nur zu relativ niedrigen Gehalten an Trockensubstanz von vorzugsweise 5 Gew.-% bis 50 Gew.-%, insbesondere von 10 Gew.-% bis 40 Gew.-% gelangt. Das Konzentrat wird einem zweckmäßigerweise zuvor hergestellten trockenen, pulverförmigen bis körnigen Gemisch der oben beschriebenen Zuschlagstoffe zudosiert. Die Zuschlagstoffe werden vorzugsweise so unter den genannten Trägermaterialien und Granulierhilfsmitteln ausgewählt, daß das entstehende Enzymextrudat ein Schüttgewicht von 700 g/l bis 1200 g/l aufweist. Der Wassergehalt der Mischung sollte so gewählt werden, daß sie sich bei der Bearbeitung mit Rühr- und Schlagwerkzeugen in körnige, bei Raumtemperatur nicht klebende Partikel überführen und bei Anwendung höherer Drücke plastisch verformen und extrudieren läßt. Das rieselfähige Vorgemisch wird im Prinzip bekannter Weise anschließend in einem Kneter sowie einem angeschlossenen Extruder zu einer plastischen Masse verarbeitet, wobei als Folge der mechanischen Bearbeitung sich die Masse auf Temperaturen zwischen 40°C und 60°C, insbesondere 45°C bis 55°C erwärmen kann. Das den Extruder verlassende Gut wird durch eine Lochscheibe mit nachfolgendem Abschlagmesser geführt und dadurch zu zylinderförmigen Partikeln definierter Größe zerkleinert. Zweckmäßigerweise beträgt der Durchmesser der Bohrungen in der Lochscheibe 0,7 mm bis 1,2 mm, vorzugsweise 0,8 mm bis 1,0 mm. Das Verhältnis zwischen Länge und Dicke des Extrudats liegt vorzugsweise im Bereich von 0,9 bis 1,1, insbesondere bei 1,0. Die in dieser Form vorliegenden Partikel können, gegebenenfalls nach einem Trocknungsschritt, direkt weiterverarbeitet werden. Es hat sich jedoch als vorteilhaft erwiesen, die den Extruder und Zerhacker verlassenden zylindrischen Partikel anschließend zu sphäronisieren, das heißt sie in geeigneten Vorrichtungen abzurunden und zu entgraten. Ein solches Sphäronisierungsverfahren ist beispielsweise in den deutschen Auslegeschriften DE 21 37 042 und DE 21 37 043 beschrieben. Man verwendet hierzu eine Vorrichtung, die aus einem zylindrischen Behälter mit stationären, festen Seitenwänden und einer bodenseitig drehbar gelagerten Reibplatte bestehen. Vorrichtungen dieser Art sind unter der Warenbezeichnung Marumerizer^{(R)} in der Technik verbreitet. Nach der Sphäronisierung werden die noch feuchten Kügelchen kontinuierlich oder chargenweise, vorzugsweise unter Verwendung einer Wirbelschichttrockenanlage, bei vorzugsweise 35 °C bis 50 °C und insbesondere bei einer maximalen Produkttemperatur von 45 °C, bis zu einem Restfeuchtegehalt von 4 Gew.-% bis 10 Gew.-%, vorzugsweise 5 Gew.-% bis 8 Gew.-% getrocknet, falls sie vorher höhere Wassergehalte aufweisen. Zweckmäßigerweise in diesem Verfahrensstadium können durch Sieben oder Windsichten bei der Herstellung des Extrudatkerns eventuell auftretende staubförmige Anteile mit einer Korngröße unter 0,1 mm, insbesondere unter 0,4 mm sowie eventuelle Grobanteile mit einer Korngröße über 2 mm, insbesondere über 1,6 mm entfernt und gegebenenfalls in den Herstellungsprozess zurückgeführt werden. Vorzugsweise wird der Extrusionsprozeß so geführt, daß die für die erfindungsgemäßen Enzymgranulate geeigneten entstehenden Extrudatkerne eine derartige Teilchengrößenverteilung aufweisen, daß weniger als 10 Gew.-%, insbesondere weniger als 2 Gew.-% der Teilchen einen Durchmesser unter 0,2 mm, 10 Gew.-% bis 20 Gew.-% der Teilchen einen Durchmesser von 0,2 mm bis unter 0,4 mm und 80 Gew.-% bis 90 Gew.-% der Teilchen einen Durchmesser von 0,4 mm bis unter 0,8 mm aufweisen.

Nach oder vorzugsweise während der Trocknung können zusätzlich Stoffe zum Umhüllen und Beschichten der Extrudatpartikel eingebracht werden. Dies kann insbesondere dann vorteilhaft sein, wenn der so hergestellte, erfindungsgemäß geeignete Extrudatkern vor seiner Weiterverarbeitung zum fertigen erfindungsgemäßen Enzymgranulat über einen längeren Zeitraum zwischengelagert werden soll. Falls sich die weiteren Verarbeitungsschritte direkt anschließen, ist das Aufbringen einer Schutzumhüllung auf den Extrudatkern in der Regel nicht erforderlich.

Das so erhaltene Kernteilchen, welches das erste Enzym enthält, wird mit einem auf beliebige Weise teilchenförmig konfektioniertem zweiten Enzym und gegebenenfalls weiteren teilchenförmig konfektionierten Enzymen unter Agglomerationsbedingungen vermischt, wobei wesentlich ist, daß die mit dem Extrudat zu vermischenden Teilchen einen derartigen mittleren Teilchendurchmesser aufweisen, daß die mittlere Teilchengröße des das erste Enzym enthaltenden Extrudats das 1,1- bis 3-fache derjenigen des zweiten oder weiteren teilchenförmig konfektionierten Enzyms beträgt. Die kleineren, das zweite Enzym oder die weiteren Enzyme enthaltenden Teilchen besitzen eine derartige Größenverteilung, daß weniger als 25 %, insbesondere weniger als 20 % der Teilchen einen Durchmesser unter 0,5 mm, von 20 % bis 60 % der Teilchen einen Durchmesser im Bereich von 0,5 mm bis 0,87 mm und 100 % der Teilchen einen Durchmesser unterhalb von 1,2 mm aufweisen. Demgegenüber weisen vorzugsweise weniger als 10 %, insbesondere weniger als 6 % der Extrudatkerne einen Durchmesser unterhalb von 0,61 mm und weniger als 10 % der Extrudatkerne einen Durchmesser über 1,23 mm, insbesondere über 1,22 mm auf.

Vorzugsweise werden die Mengenverhältnisse von Extrudatkern zu kleinerem Enzymteilchen in Abhängigkeit von den verschiedenen Teilchengrößen gewählt. So ist es erfindungsgemäß möglich, bei Extrudatkernen, welche einen 2,5-bis 3-fachen mittleren Teilchendurchmesser der kleineren Enzymteilchen aufweisen, das Gewichtsverhältnis von Extrudatkern zu kleineren Enzymteilchen im Bereich von 30:70 bis 90:10, insbesondere von 40:60 bis 60:40, einzustellen. Wenn die Extrudatkerne einen mittleren Teilchendurchmesser im Bereich vom 1,8- bis 2,2-fachen desjenigen der kleineren Enzymteilchen aufweisen, sind entsprechende Gewichtsverhältnisse von mindestens 70:30 bevorzugt.

Vorzugsweise wird die Aufbaugranulation derart durchgeführt, daß man die Mischung der die Enzyme enthaltenden Teilchen in einer Wirbelschicht mit einem üblichen Bindemittel, welches in seiner einfachsten Ausführungsform Wasser sein kann, besprüht. Geeignete Bindemittel finden sich auch unter den nichtionischen Tensiden und insbesondere unter den Filmbildnern unter den vorgenannten wasserlöslichen organischen Polymeren, beispielsweise Carboxymethylcellulose und/oder Polyethylenglykol, die in Substanz oder in insbesondere wäßriger Lösung eingesetzt werden können. Geeignete nichtionische Tenside sind insbesondere solche, welche bei Raumtemperatur fest und bei der Agglomerationstemperatur gerade so flüssig sind, daß sie in der Lage sind, die Extrudatkerne mit den kleineren Enzymteilchen zu verkleben. Weiterhin lassen sich im Agglomerationsstadium auch Farbstoffe oder Pigmente auf die Partikel aufbringen, um so eine eventuelle Eigenfarbe, die meist vom Enzymkonzentrat herrührt, zu überdecken beziehungsweise zu verändern. Als inerte und physiologisch unbedenkliche Pigmente haben sich insbesondere Titandioxid und Calciumcarbonat bewährt, die anschließend oder vorzugsweise zusammen mit dem Bindemittel in wäßriger Dispersion eingebracht werden. Das über die Pigmentdispersion beziehungsweise über das Bindemittel zugeführte Wasser wird bei der gleichzeitig vorgenommenen oder anschließend erneut erforderlichen Trocknung wieder entfernt.

Vorzugsweise wird im Verfahrensschritt des Zusammenfügens der einzelnen enzymhaltigen Teilchen zum erfindungsgemäßen Enzymgranulat als Bindemittel eine wäßrige Dispersion auf die enzymhaltigen Teilchen aufgesprüht, die 40 Gew.-% bis 75 Gew.-% Wasser, 15 Gew.-% bis 30 Gew.-% filmbildendes Polymer, welches insbesondere eine Mischung aus Polyethylenglykol mit einem mittleren Molekulargewicht von 8000 bis 15000 und Carboxymethylcellulose im Gewichtsverhältnis von 30:1 bis 50:1 ist, und 5 Gew.-% bis 30 Gew.-% Pigment, welches insbesondere eine Mischung aus Titandioxid und Calciumcarbonat im Gewichtsverhältnis von 5:1 bis 10:1 ist, enthält. Die Menge des eingebrachten Bindemittels beträgt vorzugsweise nicht über 40 Gew.-%, insbesondere 10 Gew.-% bis 35 Gew.-%, bezogen auf die Gesamtheit der enzymhaltigen Teilchen, wobei insbesondere bei Einsatz von Wasser oder wasserhaltigem Bindemittel anschließend oder vorzugsweise während des Aufbringens des wäßrigen Bindemittels auf Wassergehalte, bezogen auf entstehendes erfindungsgemäßes Enzymgranulat, von 5 Gew.-% bis 10 Gew.-%, insbesondere von 7 bis 9 Gew.-% getrocknet wird.

Eine weitere Ausführungsform des erfindungsgemäßen Verfahrens besteht darin, nicht sämtliche größeren Extrudatkerne mit den kleineren Enzymteilchen zu umhüllen, sondern nur einen Teil, wenn dies für den gewünschten Anwendungszweck des entstehenden Gemischs aus erfindungsgemäßem Enzymgranulat und nicht mit zweitem Enzym umhüllten Extrudatkern zu genügenden Aktivitäten an zweitem Enzym führt.

Das erfindungsgemäße Enzymgranulat wird vorzugsweise zur Herstellung fester, insbesondere teilchenförmiger Wasch- oder Reinigungsmittel verwendet, die durch einfaches Vermischen der Enzymgranulate mit in derartigen Mitteln üblichen weiteren Pulverkomponenten erhalten werden können. Ein bevorzugter Einsatzbereich insbesondere erfindungsgemäßer Protease-Amylase-Granulate liegt auf dem Gebiet der Geschirrreinigungsmittel für die maschinelle Anwendung, welche vorzugsweise als verdichtete Pulver mit erhöhten Schüttgewichten im Bereich von bevorzugt 750 bis 1 000 g/l oder in Tablettenform angeboten werden. Zur Herstellung derartiger Tabletten geht man vorzugsweise derart vor, daß man das erfindungsgemäße Enzymgranulat mit allen weiteren Bestandteilen in einem Mischer vermischt und das Gemisch mittels herkömmlicher Tablettenpressen, beispielsweise Exzenterpressen oder Rundläuferpressen, mit Preßdrucken im Bereich von 200 · 10⁵ Pa bis 1 500 · 10⁵ Pa verpresst. Man erhält so problemlos bruchfeste und dennoch unter Anwendungsbedingungen ausreichend schnell lösliche Tabletten mit Biegefestigkeiten von normalerweise über 150 N. Vorzugsweise weist eine derart hergestellte Tablette ein Gewicht von 15 g bis 40 g, insbesondere von 20 g bis 30 g, bei einem Durchmesser von 35 mm bis 40 mm auf.

Für die Einarbeitung in teilchenförmige Wasch- und Reinigungsmittel weist das Enzymgranulat vorzugsweise mittlere Korngrößen im Bereich von 0,9 mm bis 1,8 mm, insbesondere von 1,0 mm bis 1,5 mm auf.Die erfindungsgemäßen Granulate enthalten vorzugsweise weniger als 5 Gew.-%, insbesondere höchstens 1 Gew.-% an Partikeln mit Korngrößen außerhalb des Bereichs von 0,2 mm bis 1,6 mm.

Die erfindungsgemäß erhaltene Enzymzubereitung besteht aus weitgehend abgerundeten, staubfreien Partikeln, die in der Regel ein Schüttgewicht von etwa 650 bis 1050 Gramm pro Liter, insbesondere 700 bis 880 Gramm pro Liter aufweisen. Die genannten Schüttgewichte entsprechen in der Regel denjenigen der Ausgangspartikel. Die erfindungsgemäßen Granulate zeichnen sich durch eine sehr hohe Lagerstabilität, insbesondere bei Temperaturen über Raumtemperatur und hoher Luftfeuchtigkeit, aus, die obwohl miteinander reagieren könnende Enzyme vorhanden sind, nicht die Lagerstabilität der separat gelagerten Bestandteile Extrudatkern und kleineres Enzymteilchen unterschreitet. Dies gilt sowohl für die erfindungsgemäßen Enzymgranulate bzw. deren Bestandteile als solche wie auch für die in teilchenförmige Wasch- oder Reinigungsmittel eingearbeiteten erfindungsgemäßen Enzymgranulate bzw. deren Bestandteile. Als weiterer Vorteil der erfindungsgemäßen Enzymgranulate ist ihr rasches Lösungsverhalten unter Anwendungsbedingungen in der Waschflotte zu bemerken. Vorzugsweise setzen die erfindungsgemäßen Granulate 100 % ihrer Enzymaktivität innerhalb von 3 Minuten, insbesondere innerhalb von 70 Sekunden bis 2 Minuten, in Wasser bei 25 °C frei.

### Beispiele

### Beispiel 1

Durch Fermentation von nach dem in der internationalen Patentanmeldung WO 91/2792 beschriebenen Verfahren durch Transformation einer Gensequenz aus Bacillus lentus DSM 5483 modifiziertem Bacillus licheniformis (ATCC 53926) analog dem in der deutschen Patentschrift DE 29 25 427 angegebenen Verfahren wurde eine biomassehaltige Fermenterbrühe erhalten, die ca. 65 000 Proteaseeinheiten pro Gramm (PE/g) enthielt. Diese wurde durch Dekantieren, Querstrom-Mikrofiltration, Ultrafiltation (Trenngrenze bei Molekulargewicht 10 000) und anschließendes Eindampfen im Vakuum gemäß dem in der internationalen Patentanmeldung WO 92/11347 beschriebenen Vorgehen zu einem Proteasegehalt von 700 000 PE/g aufkonzentriert. Die so aufkonzentrierte Fermenterbrühe wurde in einem mit rotierendem Schlagwerkzeug ausgerüsteten Mischer mit den in Tabelle 1 aufgeführten Zuschlägen vermischt und in einem mit einer Außenkühlung versehenen Kneter homogenisiert. Die Extrusion der plastischen Masse erfolgte mittels eines mit einer Lochscheibe (Lochdurchmesser 0,9 mm) und einem rotierenden Messer ausgerüsteten Extruder. Man erhielt die in Tabelle 1 durch ihre Zusammensetzung charakterisierten Extrudatkerne **X1** bis **X4** mit Längen von jeweils 0,9 mm, die in einer Sphäronisierungsvorrichtung (Marumerizer^{(R)}) während einer Bearbeitungszeit von etwa 1 Minute zu gleichmäßig abgerundeten Partikeln verformt und entgratet wurden. Das den Sphäronisator verlassende Gut wurde in einem Wirbelschichttrockner bei Temperaturen von 40 °C bis 45 °C innerhalb von 15 Minuten auf einen Wassergehalt von 6 Gew.-% getrocknet. Durch anschließendes Sieben wurden Partikel mit Teilchengrößen unter 0,4 mm und über 1,6 mm (Mengenanteil 1,2 Gew.-%) entfernt, die dem Prozeß auf der Stufe des Vermischens mit den Zuschlagstoffen wieder zugeführt wurden.

**Tabelle 1:**

| Zusammensetzung der Extrudate [Gew.-%] | | | | |
|---|---|---|---|---|
| | **X1** | **X2** | **X3** | **X4** |
| Fermenterbrühe | 34 | 32 | 32 | 32 |
| Zellulosepulver^{a)} | 4,5 | 4,5 | 4,5 | 4,5 |
| Saccharose | 3,5 | 3,5 | 3,5 | 3,5 |
| Weizenmehl T 450 | 19 | 19 | 19 | 19 |
| Maisstärke | 37 | 38 | 39 | 36 |
| CMC-I^{b)} | 2 | 2 | - | 3 |
| CMC-II^{c)} | - | - | 1 | - |
| PEG^{d)} | - | 1 | - | 2 |
| Tensid^{e)} | - | - | 1 | - |

| | | | | |
|---|---|---|---|---|
| ^{a)}: Technocel^{(R)} 30 (Hersteller Cellulose Füllstoff Fabrik) | | | | |
| ^{b)}: Carbocel^{(R)} 300 (Substitutionsgrad 0,65-0,75; Hersteller Lamberti CMC) | | | | |
| ^{c)}: Carbocel^{(R)} 500 (Substitutionsgrad 0,85-0,95; Hersteller Lamberti CMC | | | | |
| ^{d)}: Polyethylenglykol, mittleres Molekulargewicht 400 | | | | |
| ^{e)}: 40-fach ethoxylierter Talgfettalkohol (Hersteller Henkel) | | | | |

Die Extrudatkerne **X1**, **X2**, **X3** bzw. **X4** (mittlere Teilchengröße jeweils 918 µm) wurden mit einem Lipasegranulat (Lipolase^{(R)}, Hersteller Novo, mittlere Teilchengröße 668,5 µm, Lipaseaktivität 100 000 LU/g) im Gewichtsverhältnis 3,3 zu 1 vermischt. In einem Wirbelschicht-Sprühgranulator des Typs STREA-1 der Fa. Aeromatic wurde das Protease-Lipase Gemisch in der Wirbelschicht gecoated. Die Coatingsuspension bestand aus:

| | |
|---|---|
| Titandioxid | 17 % |
| Carboxymethylcellulose | 0,5 % |
| Kalziumcarbonat | 2,5 % |
| Polyethylenglykol mit einem mittleren Molekulargewicht von 12.000 | 19 % |
| Wasser | 61 %. |

Während des Aufsprühens der wäßrigen Coatingsuspension agglomerierten die feineren Lipasegranulate an den grobkörnigen Proteaseextrudaten. Während des Coatens wurden zur gleichzeitigen Trocknung folgende Betriebsparameter eingestellt:

| | |
|---|---|
| Produkttemperatur | 36 °C |
| Zulufttemperatur | 47 °C |
| Ablufttemperatur | 33 °C. |

Nach dem Aufsprühen von 28,8 Gew.-%, bezogen auf vorgelegtes Enzymgemisch, der oben aufgeführten Coatingsuspension waren die Granulate gleichmäßig von der Farb- und Schutzschicht umhüllt. Die Lipaseteilchen waren dabei mit den Proteasegranulaten vereinigt worden und lagen gleichmäßig verteilt im Endprodukt des Enzymgemisches vor. Die Löslichkeit des Enzymgemisches betrug 1 Min. und 26 Sek. Die Versuche zur Lagerstabilität ergaben keinerlei Änderung zu Lagerversuchen von separat gelagerten Lipase- bzw. Proteasegranulaten. Ebenso konnte keine Erhöhung des Staubabriebs festgestellt werden.

### Beispiel 2

Wie in Beispiel 1 beschrieben wurden die Extrudatkerne X1, X2, X3 bzw. X4 mit einem Amylasegranulat (Maxamyl^{(R)} CXT 5000, Hersteller Gist Brocades, mittlere Teilchengröße 306 µm) im Gewichtsverhältnis 1,1 zu 1 vermischt und gecoated. Die Coatingsuspension bestand aus:

| | |
|---|---|
| Titandioxid | 17 % |
| Stearylalkohol | 19 % |
| Eumulgin^{(R)} RT 40^{a)} | 3 % |
| Wasser | 61 %. |

| | |
|---|---|
| a) 40-fach ethoxyliertes Ricinusöl (Hersteller Henkel KGaA) | |

Während des Aufsprühens der wäßrigen Coatingsuspension verdampfte das Wasser erst nach dem Auftreffen auf das Granulatkorn vollständig. Solange die Coatingsuspension auf dem Granulatkorn noch fließfähig war, hafteten die feinen Amylasegranulate auf der Oberfläche der größeren Proteasegranulate an. Nach der Verdampfung des Wassers aus der Coatingsuspension bildete die Coatingschicht eine feste Verbindung zwischen den verschiedenen Enzymgranulaten. Außerdem wurde das Enzymagglomerat von der schützenden Coatingschicht überzogen.

Die Amylasegranulate lagen in dem Enzymgemisch homogen verteilt vor. Die Lagerbeständigkeit, Löslichkeit bzw. der Staubabrieb unterschied sich nicht von den Produkten, die nur aus einer Enzymart bestehen.

### Beispiel 3:

Es wurde wie in Beispiel 2 gearbeitet. Das Amylasegranulat haftete in diesem Fall durch das Aufsprühen eines nichtionischen Tensids mit niedrigem Fließpunkt an dem Proteasegranulat an. Als Niotensid wurde ein Fettalkohol C₁₆-C₁₈-5E0 (Dehydol^{(R)} TA5, Hersteller Henkel KGaA) verwendet. Der Fließpunkt des Tensides betrug ca. 35 °C. Die Produkttemperatur in der Wirbelschicht lag bei 35 bis 39 °C. Bei diesen Temperaturen war das Niotensid noch fließfähig und konnte sich gleichmäßig auf dem gesamten Enzymgranulat verteilen. Da im Bereich des Fließpunkts des Niotensids gearbeitet wurde, war das Tensid bereits zähflüssig und die Amylase blieb an den einzelnen Proteasegranulaten "kleben". Es wurden 50 g Niotensid pro kg Enzymteilchen aufgesprüht.

Die Agglomerate wurden im Anschluß von einer aufgesprühten Coatingschmelze umhüllt und damit geschützt sowie gleichmäßig weiß eingefärbt.

Für 1 kg der Enzymgranulate wurden 11,4 % der Coatingschmelze verwendet. Die Coatinghülle setzte sich, bezogen auf entstehendes Fertigprodukt, wie folgt zusammen:

| | |
|---|---|
| Stearylalkohol | 5,4 % |
| Eumulgin^{(R)} RT 40 | 1,3 % |
| Titandioxid | 4,7 %. |

### Beispiel 4:

Unter Beibehaltung der sonstigen Verfahrensparameter des Beispiels 3 wurde eine Schmelze (Temperatur ca. 40 °C) aus 67 Gew.-% technischem Stearylalkohol, 10 Gew.-% 40-fach ethoxyliertem Fettalkohol (Disponil^{(R)} TA 430, Hersteller Henkel KGaA) und 23 Gew.-% Titandioxid auf ein vorgelegtes Gemisch (Gewichtsverhältnis 2:1:1) aus Proteaseextrudat X1, Lipasegranulat (Lipolase^{(R)} wie in Beispiel 1) und Cellulasegranulat (Celluzyme^{(R)} 0.7 T, Hersteller Novo Nordisk; mittlere Teilchengröße ca. 500 µm) aufgesprüht. Die kleineren Lipase- und Cellulasegranulate agglomerierten an das Proteaseextrudat und das entstehende Dreienzymgranulat wurde durch die weiter aufgesprühte Schmelze umhüllt und damit geschützt sowie gleichmäßig weiß eingefärbt. Bezogen auf entstehendes Mehrenzymgranulate wurden 21,5 % der Coatingschmelze verwendet.

## Patentansprüche

1. Verfahren zur Herstellung von Enzymgranulaten mit mittleren Teilchengrößen von 1 mm bis 2,2 mm, welche mindestens zwei verschiedene Enzyme enthalten, gekennzeichnet durch Extrudieren eines durch Vermischen einer gegegebenfalls von unlöslichen Bestandteilen befreiten und aufkonzentrierten Fermentationsbrühe eines ersten Enzyms mit organischem und/oder anorganischem Trägermaterial und gegebenenfalls Granulierhilfsmittel als Zuschlagstoffen entstandenen Enzym-Vorgemischs, gegebenenfalls Sphäronisierung des Extrudats in einem Rondiergerät, Trocknung bis zu einem Restfeuchtegehalt von 4 Gew.-% bis 10 Gew.-%, falls das Extrudat vorher höhere Wassergehalte aufweist, Vermischen mit einem teilchenförmig konfektioniertem zweiten Enzym und gegebenenfalls weiteren teilchenförmig konfektionierten Enzymen, wobei weniger als 25 Gew.-% der Teilchen einen Durchmesser unter 0,5 mm aufweisen, unter Agglomerationsbedingungen, wobei gegebenenfalls ein Bindemittel zum Einsatz kommt, und gegebenenfalls Aufbringen eines Farbstoff oder Pigment enthaltenden Überzugs, wobei die mittlere Teilchengröße des das erste Enzym enthaltenden Extrudatkerns das 1,1-bis 3-fache derjenigen des zweiten oder weiteren teilchenförmig konfektionierten Enzyms beträgt.

2. Verfahren nach Anspruch 1, dadurch gekennzeichnet, daß die mittlere Teilchengröße des das erste Enzym enthaltenden Extrudatkerns das 1,1-bis 2,5-fache, insbesondere das 1,2- bis 1-fache derjenigen des zweiten oder weiteren teilchenförmig konfektionierten Enzyms beträgt.

3. Verfahren nach Anspruch 1 oder 2, dadurch gekennzeichnet, daß der Extrudatkern Protease enthält und mindestens ein weiteres teilchenförmig konfektioniertes Enzym, ausgewählt aus Lipase, Amylase, Cellulase oder Oxidase, unter Agglomerationsbedingungen aufgebracht wird.

4. Verfahren nach Anspruch 1 oder 2, dadurch gekennzeichnet, daß der Extrudatkern Lipase enthält und mindestens ein weiteres teilchenförmig konfektioniertes Enzym, ausgewählt aus Protease, Amylase, Cellulase oder Oxidase, unter Agglomerationsbedingungen aufgebracht wird.

5. Verfahren nach einem der Ansprüche 1 bis 4, dadurch gekennzeichnet, daß man die Agglomeration unter Aufsprühen einer wäßrigen Dispersion als Bindemittel durchführt, die 40 Gew.-% bis 75 Gew.-% Wasser, 15 Gew.-% bis 30 Gew.-% filmbildendes Polymer und 5 Gew.-% bis 30 Gew.-% Pigment enthält.

6. Verfahren nach Anspruch 5, dadurch gekennzeichnet, daß die Menge des eingebrachten Bindemittels nicht über 40 Gew.-%, insbesondere 10 Gew.-% bis 35 Gew.-%, bezogen auf die Gesamtheit der enzymhaltigen Teilchen, beträgt.

7. Verfahren nach Anspruch 5 oder 6, dadurch gekennzeichnet, daß man anschließend oder insbesondere während des Aufbringens des wäßrigen Bindemittels auf Wassergehalte, bezogen auf entstehendes Enzymgranulat, von 5 Gew.-% bis 10 Gew.-% trocknet.

8. Verfahren nach einem der Ansprüche 5 bis 7, dadurch gekennzeichnet, daß man als filmbildendes Polymer im Bindemittel eine Mischung aus Polyethylenglykol mit einem mittleren Molekulargewicht von 8000 bis 15000 und Carboxymethylcellulose im Gewichtsverhältnis von 30:1 bis 50:1 einsetzt.

9. Verfahren nach einem der Ansprüche 5 bis 8, dadurch gekennzeichnet, daß man als Pigment eine Mischung aus Titandioxid und Calciumcarbonat im Gewichtsverhältnis von 5:1 bis 10:1 einsetzt.

10. Für die Einarbeitung in Wasch- oder Reinigungsmittel geeignetes Enzymgranulat, enthaltend Enzym und anorganisches und/oder organisches Trägermaterial sowie gegebenenfalls Granulierhilfsmittel, dadurch gekennzeichnet, daß es aus einem extrudierten, ein erstes Enzym enthaltenden Kern, an den Teilchen, welche ein zweites Enzym und gegebenenfalls Teilchen, welche weitere Enzyme enthalten, agglomeriert sind, wobei weniger als 25 Gew.-% der Teilchen einen Durchmesser unter 0,5 mm aufweisen, und einem aufgesprühten Außencoating besteht.

11. Enzymgranulat nach Anspruch 10, dadurch gekennzeichnet, daß es Protease im Extrudatkern und Lipase in den agglomerierten Teilchen oder Lipase im Extrudatkern und Protease in den agglomerierten Teilchen enthält.

12. Enzymgranulat nach Anspruch 11, dadurch gekennzeichnet, daß es zusätzlich agglomerierte Teilchen enthält, welche Cellulase enthalten.

13. Enzymgranulat nach Anspruch 10, dadurch gekennzeichnet, daß es Protease im Extrudatkern und Amylase in den agglomerierten Teilchen oder Amylase im Extrudatkern und Protease in den agglomerierten Teilchen enthält.

14. Enzymgranulat nach einem der Ansprüche 10 bis 13, dadurch gekennzeichnet, daß es ein Schüttgewicht von 650 g/l bis 1050 g/l aufweist.

## Claims

1. A process for the production of enzyme granules with average particle sizes of 1 mm to 2.2 mm which contain at least two different enzymes, characterized by extrusion of an enzyme compound formed by mixing a fermentation broth of a first enzyme - optionally freed from insoluble constituents and concentrated - with organic and/or inorganic carrier material and optionally granulation aids as additives, optionally spheronizing the extrudate in a spheronizer, drying to a residual moisture content of 4% by weight to 10% by weight if the extrudate previously had higher water contents, mixing with a particulate second enzyme and optionally other particulate enzymes, less than 25% of the particles being smaller than 0.5 mm in diameter, under agglomeration conditions, a binder optionally being used, and optionally applying a dye- or pigment-containing coating, the average particle size of the extrudate core containing the first enzyme being 1.1 to 3 times the average particle size of the second particulate enzyme or further particulate enzymes.

2. A process as claimed in claim 1, characterized in that the average particle size of the extrudate core containing the first enzyme is 1.1 to 2.5 times and, more particularly, 1.2 to 1 times that of the second particulate enzyme or other particulate enzymes.

3. A process as claimed in claim 1 or 2, characterized in that the extrudate core contains protease and at least one other particulate enzyme selected from lipase, amylase, cellulase or oxidase is applied under agglomeration conditions.

4. A process as claimed in claim 1 or 2, characterized in that the extrudate core contains lipase and at least one other particulate enzyme selected from protease, amylase, cellulase or oxidase is applied under agglomeration conditions.

5. A process as claimed in any of claims 1 to 4, characterized in that agglomeration is carried out by spray application as binder of a mixture containing 40% by weight to 75% by weight of water, 15% by weight to 30% by weight of film-forming polymer and 5% by weight to 30% by weight of pigment.

6. A process as claimed in claim 5, characterized in that the quantity of binder introduced is not more than 40% by weight and, more particularly, is from 10% by weight to 35% by weight, based on all the enzyme-containing particles.

7. A process as claimed in claim 5 or 6, characterized in that application of the water-containing binder is followed or, more particularly, accompanied by drying to water contents, based on the enzyme granules formed, of 5% by weight to 10% by weight.

8. A process as claimed in any of claims 5 to 7, characterized in that a mixture of polyethylene glycol with an average molecular weight of 8,000 to 15,000 and carboxymethyl cellulose in a ratio by weight of 30:1 to 50:1 is used as the film-forming polymer in the binder.

9. A process as claimed in any of claims 5 to 8, characterized in that a mixture of titanium dioxide and calcium carbonate in a ratio by weight of 5:1 to 10:1 is used as the pigment.

10. Enzyme granules suitable for incorporation in detergents or cleaning compositions and containing enzyme and inorganic and/or organic carrier material and optionally granulation aids, characterized in that they consist of an extruded core containing a first enzyme onto which particles containing a second enzyme and optionally particles containing further enzymes are agglomerated, less than 25% of the particles being smaller than 0.5 mm in diameter, and of an outer coating applied by spraying.

11. Enzyme granules as claimed in claim 10, characterized in that they contain protease in the extrudate core and lipase in the agglomerated particles or lipase in the extrudate core and protease in the agglomerated particles.

12. Enzyme granules as claimed in claim 11, characterized in that they additionally contain agglomerated particles containing cellulase.

13. Enzyme granules as claimed in claim 10, characterized in that they contain protease in the extrudate core and amylase in the agglomerated particles or amylase in the extrudate core and protease in the agglomerated particles.

14. Enzyme granules as claimed in any of claims 10 to 13, characterized in that they have an apparent density of 650 g/l to 1050 g/l.

## Revendications

1. Procédé de fabrication de granulés enzymatiques avec une grosseur de particule moyenne de 1 à 2,2 mm, qui renferment au moins deux enzymes différentes, caractérisé par l'extrusion d'un prémélange d'enzymes obtenu par mélangeage d'un bouillon de fermentation éventuellement débarrassé des constituants insolubles et concentré d'une première enzyme avec une matière porteuse organique et/ou inorganique et, le cas échéant, avec un adjuvant de granulation, comme agrégats, éventuellement sphéronisation du produit d'extrusion dans un appareil à arrondir, séchage jusqu'à une concentration en humidité résiduelle de 4 à 10 % en poids, au cas où le produit d'extrusion présenterait préalablement des teneurs en eau plus élevées, mélangeage avec une seconde enzyme formulée sous la forme de particules et, éventuellement, avec d'autres enzymes supplémentaires formulées sous la forme de particules, moins de 25 % en poids des particules présentant un diamètre inférieur à 0,5 mm, dans des conditions d'agglomération dans lesquelles un liant est utilisé le cas échéant, et éventuellement application d'un revêtement contenant un colorant ou un pigment, la grosseur moyenne des particules du noyau de produit d'extrusion renfermant la première enzyme atteignant 1,1 à 3 fois celle de la seconde enzyme ou des autres enzymes supplémentaires formulée(s) sous la forme de particules.

2. Procédé selon la revendication 1, caractérisé en ce que la grosseur de particule moyenne du noyau de produit d'extrusion contenant la première enzyme atteint 1,1 à 2,5 fois, en particulier 1,2 à 1 fois celle de la seconde enzyme ou des autres enzymes supplémentaires formulée(s) sous la forme de particules.

3. Procédé selon la revendication 1 ou 2, caractérisé en ce que le noyau du produit d'extrusion contient de la protéase et qu'au moins une autre enzyme formulée sous la forme de particules, sélectionnée parmi la lipase, l'amylase, la cellulase ou l'oxydase, est appliquée sur ledit produit d'extrusion dans des conditions d'agglomération.

4. Procédé selon la revendication 1 ou 2, caractérisé en ce que le noyau du produit d'extrusion contient de la lipase et qu'au moins une autre enzyme formulée sous la forme de particules, sélectionnée parmi la protéase, l'amylase, la cellulase et l'oxydase, est appliquée sur ledit produit d'extrusion dans des conditions d'agglomération.

5. Procédé selon une des revendications 1 à 4, caractérisé en ce que l'on opère l'agglomération en pulvérisant comme liant une dispersion aqueuse, qui contient 40 à 75 % en poids d'eau, 15 à 30 % en poids de polymère filmogène et 5 à 30 % en poids de pigment.

6. Procédé selon la revendication 5, caractérisé en ce que la quantité du liant incorporé n'est pas supérieure à 40 % en poids, en étant comprise, en particulier, entre 10 et 35 % en poids, par rapport à la totalité des particules enzymaiques.

7. Procédé selon la revendication 5 ou 6, caractérisé en ce que l'on sèche après ou pendant l'application du liant aqueux jusqu'à des teneurs en eau de 5 à 10 % en poids, par rapport au granulé enzymatique obtenu.

8. Procédé selon une des revendications 5 à 7, caractérisé en ce que l'on utilise comme polymère filmogène dans le liant, un mélange de polyéthylèneglycol avec un poids moléculaire moyen de 8000 à 15 000 et de carboxyméthylcellulose, dans un rapport pondéral de 30:1 à 50:1.

9. Procédé selon une des revendications 5 à 8, caractérisé en ce que l'on utilise comme pigment, un mélange de dioxyde de titane et de carbonate de calcium, dans un rapport pondéral de 5:1 à 10:1.

10. Granulé enzymatique approprié pour l'incorporation dans des agents de lavage ou de nettoyage, contenant une enzyme et une matière porteuse inorganique/et ou organique, ainsi que, le cas échéant, un adjuvant de granulation, qui est caractérisé en ce qu'il est constitué d'un noyau extrudé contenant une première enzyme auquel sont agglomérées des particules, qui renferment une seconde enzyme et, éventuellement, des particules, qui contiennent d'autres enzymes supplémentaires, moins de 25 % en poids des particules présentant un diamètre inférieur à 0,5 mm, et d'une enduction extérieure pulvérisée

11. Granulé enzymatique selon la revendication 10, caractérisé en ce qu'il contient de la protéase dans le noyau du produit d'extrusion et de la lipase dans les particules agglomérées, ou de la lipase dans le noyau du produit d'extrusion et de la protéase dans les particules agglomérées.

12. Granulé enzymatique selon la revendication 11, caractérisé en ce qu'il contient en plus des particules agglomérées, qui renferment de la cellulase.

13. Granulé enzymatique selon la revendication 10, caractérisé en ce qu'il contient de la protéase dans le noyau du produit d'extrusion et de l'amylase dans les particules agglomérées ou de l'amylase dans le noyau du produit d'extrusion et de la protéase dans les particules agglomérées.

14. Granulé enzymatique selon une des revendication 10 à 13, caractérisé en ce qu'il présente une densité en vrac de 650 à 1050 g/l.
